(19) Europäisches Patentamt
European Patent Office
Office européen des brevets

(11) **EP 2 504 368 B1**

(12) **EUROPÄISCHE PATENTSCHRIFT**

(45) Veröffentlichungstag und Bekanntmachung des Hinweises auf die Patenterteilung:
**08.01.2014  Patentblatt 2014/02**

(51) Int Cl.:
*C08F 220/06* (2006.01)  *C08J 3/12* (2006.01)
*C08F 2/44* (2006.01)

(21) Anmeldenummer: **10776704.8**

(22) Anmeldetag: **12.11.2010**

(86) Internationale Anmeldenummer:
**PCT/EP2010/067382**

(87) Internationale Veröffentlichungsnummer:
**WO 2011/061125 (26.05.2011 Gazette 2011/21)**

(54) **VERFAHREN ZUR HERSTELLUNG WASSERABSORBIERENDER POLYMERPARTIKEL MIT VERBESSERTER FARBSTABILITÄT**

METHOD FOR PRODUCING WATER-ABSORBING POLYMER PARTICLES HAVING IMPROVED COLOR STABILITY

PROCÉDÉ POUR PRODUIRE DES PARTICULES POLYMÈRES HYDROABSORBANTES PRÉSENTANT UNE STABILITÉ DE COULEUR AMÉLIORÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.11.2009   US 263419 P**

(43) Veröffentlichungstag der Anmeldung:
**03.10.2012   Patentblatt 2012/40**

(73) Patentinhaber: **BASF SE**
**67056 Ludwigshafen (DE)**

(72) Erfinder:
• **HERFERT, Norbert**
  **63674 Altenstadt (DE)**
• **DANIEL, Thomas**
  **67165 Waldsee (DE)**

(56) Entgegenhaltungen:
**WO-A1-2009/060062     DE-A1- 10 138 150**

**EP 2 504 368 B1**

**Beschreibung**

Die vorliegende Erfindung betrifft Verfahren zur Herstellung wasserabsorbierender Polymerpartikel, wobei mindestens ein aliphatischer Aldehyd oder dessen Umsetzungsprodukt mit einem aliphatischen Alkohol, aliphatischen Amin, Ammoniak, einem Hypophosphit oder einem Phosphit zugesetzt wird, sowie die gemäß dem erfindungsgemäßen Verfahren erhältlichen wasserabsorbierenden Polymerpartikel.

Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymer-partikel werden auch als Superabsorber bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Die Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm$^2$ (AUL0.3psi) durchläuft ein Maximum.

Zur Verbesserung der Anwendungseigenschaften, wie beispielsweise Permeabilität des gequollenen Gelbetts (SFC) in der Windel und Absorption unter einem Druck von 49.2 g/cm$^2$ (AUL0.7psi), werden wasserabsorbierende Polymerpartikel im allgemeinen oberflächennachvernetzt. Dadurch steigt der Vernetzungsgrad der Partikeloberfläche, wodurch die Absorption unter einem Druck von 49,2 g/cm$^2$ (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet, thermisch oberflächennachvernetzt und getrocknet. Dazu geeignete Vernetzer sind Verbindungen, die mit mindestens zwei Carboxylatgruppen der wasserabsorbierenden Polymerpartikel kovalente Bindungen bilden können.

Ein bei wasserabsorbierenden Polymerpartikeln oft auftretendes Problem sind Verfärbungen, die beim Lagern unter höherer Temperatur oder höherer Luftfeuchtigkeit auftreten. Derartige Bedingungen treten oft bei Lagerung in tropischen oder subtropischen Ländern auf. Unter solchen Bedingungen neigen wasserabsorbierende Polymerpartikel zum Vergilben, sie können sogar braune oder gar fast schwarze Färbung annehmen. Diese Verfärbung des eigentlich farblosen wasserabsorbierenden Polymerpartikeln ist unansehnlich und unerwünscht, da sie insbesondere bei den erwünschten dünnen Hygieneprodukten sichtbar ist und Verbraucher unansehnliche Hygieneprodukte ablehnen. Die Ursache für die Verfärbung ist nicht vollständig geklärt, allerdings scheinen reaktive Verbindungen wie Restmonomere aus der Polymerisation, die Verwendung mancher Initiatoren, Verunreinigungen des Monomeren oder des Neutralisationsmittels, Oberflächennachvernetzer oder Stabilisatoren der verwendeten Monomere eine Rolle zu spielen.

Gemäß WO 00/55245 A1 kann die Farbstabilität wasserabsorbierender Polymerpartikel durch Zusatz anorganischer Reduktionsmittel verbessert werden. Die anorganischen Reduktionsmittel können beispielsweise nach der Polymerisation dem Polymergel oder nach der thermischen Oberflächennachvernetzung zugesetzt werden.

WO 2006/058682 A1 lehrt, dass die Anwesenheit von Sauerstoff bei der thermischen Oberflächennachvernetzung zu Verfärbungen führt.

Gemäß WO 2004/084962 A1 wirkt sich die Verwendung von Sulfinsäuren als Polymerisationsinitiatoren günstig auf die Farbstabilität der erhaltenen wasserabsorbierenden Polymerpartikel aus.

WO 2008/092842 A1 und WO 2008/092843 A1 offenbaren die Beschichtung mit basischen Salzen zum selben Zweck.

Gemäß WO 2009/060062 A1 kann die Farbstabilität wasserabsorbierender Polymerpartikel mit Sulfonsäuren und deren Salzen erhöht werden, wobei die Sulfonsäuren bzw. deren Salze vorzugsweise unmittelbar vor der Oberflächennachvernetzung zugesetzt werden.

WO 03/014172 A2 beschreibt ein Verfahren zur Herstellung wasserabsorbierender Polymerpartikel, wobei die verwendete Acrylsäure vorher mit einem Aldehydfänger behandelt wurde, da insbesondere die Anwesenheit von Aldehyden zu Verfärbungen führen soll.

Aufgabe der vorliegenden Erfindung war die Bereitstellung eines Verfahrens zur Herstellung wasserabsorbierender Polymerpartikel mit verbesserter Farbstabilität. Gleichzeitig sollten die wasserabsorbierenden Polymerpartikel, insbesondere bei längerer Lagerung in warmer und feuchter Umgebung, keine unangenehmen Gerüche entwickeln.

Gelöst wurde die Aufgabe durch ein Verfahren zur Herstellung wasserabsorbierender Polymerpartikel durch Polymerisation einer Monomerlösung oder -suspension, enthaltend

a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und

e) optional ein oder mehrere wasserlösliche Polymere,

umfassend die Schritte Polymerisation der Monomerlösung zu einem Polymergel i), optional Zerkleinerung des erhaltenen Polymergels ii), Trocknung des Polymergels iii), Mahlung und Klassierung des getrockneten Polymergels zu Polymerpartikeln iv), und optional thermischer Oberflächennachvernetzung der klassierten Polymerpartikel v), dadurch gekennzeichnet, dass vor, während oder nach einem der Schritte i) bis v) mindestens ein aliphatischer Aldehyd oder dessen Umsetzungsprodukt mit einem aliphatischen Alkohol, einem aliphatischen Amin, Ammoniak, einem Hypophosphit oder einem Phosphit zugesetzt wird.

Vorzugsweise wird der aliphatische Aldehyd oder dessen Umsetzungsprodukt mit einem aliphatischen Alkohol, einem aliphatischen Amin, Ammoniak, einem Hypophosphit oder einem Phosphit nach Schritt iv) und vor, während oder nach Schritt v) zugesetzt. Ganz besonders bevorzugt wird der aliphatische Aldehyd oder dessen Umsetzungsprodukt mit einem aliphatischen Alkohol, einem aliphatischen Amin, Ammoniak, einem Hypophosphit oder einem Phosphit nach Schritt v) zugesetzt.

Aliphatische Aldehyde, Alkohole bzw. Amine im Sinne dieser Erfindung sind Verbindungen, die in ihrer Struktur keine aromatischen Ringe oder Ringsysteme aufweisen. Die erfindungsgemäß einsetzbaren Aldehyde umfassen auch di- und polyfuntionelle Aldehyde.

Als aliphatische Aldehyde werden vorzugsweise $C_1$-bis $C_5$-Aldehyde, bevorzugt $C_1$-bis $C_4$-Aldehyde, besonders bevorzugt $C_1$-bis $C_3$-Aldehyde, ganz besonders bevorzugt $C_2$-bis $C_3$-Aldehyde, eingesetzt.

Geeignete aliphatische Aldehyde sind Formaldehyd, Acetaldehyd, Propionaldehyd, Butyraldehyd, Glykolaldehyd, Glycerinaldehyd und Glyoxylsäure.

Ganz besonders bevorzugte aliphatische Aldehyde sind Glyoxylsäure und deren Salze, beispielsweise Natriumglyoxylat und Kaliumglyoxylat.

Geeignete Umsetzungsprodukte der aliphatischen Aldehyde mit aliphatischen Alkoholen sind Halbacetale und Acetale.

Geeignete Halbacetale sind dimere α-Hydroxyaldehyde, wie 2,5-Dihydroxy-1,4-dioxan, 3,6-Dihydroxy-2,5-dihydroxymethyl-1,4-dioxan. Die Halbacetale entstehen hierbei durch Addition der Hydroxylgruppe des einen α-Hydroxyaldehyds an die Aldehydgruppe des anderen α-Hydroxyaldehyds, d.h. der α-Hydroxyaldehyd ist gleichzeitig aliphatischer Aldehyd und aliphatischer Alkohol.

Geeignete Acetale sind die Acetale von Formaldehyd, Acetaldehyd, Propionaldehyd, Butyraldehyd, Glykolaldehyd, Glycerinaldehyd und Glyoxylsäure.

Ganz besonders bevorzugte Acetale sind die Acetale der Glyoxylsäure und deren Salze, wie Natrium-2,2-dimethoxyacetat, Kalium-2,2-dimethoxyacetat, Natrium-2,2-di-ethoxyacetat und Kalium-2,2-diethoxyacetat.

Geeignete Umsetzungsprodukte der aliphatischen Aldehyde mit aliphatischen Aminen sind Azomethine, Enamine und Aminale. Azomethine aus aliphatischen Aldehyden und aliphatischen Aminen polymerisieren leicht und können auch in polymerer Form eingesetzt werden.

Die Umsetzungsprodukte der aliphatischen Aldehyde mit Ammoniak können ebenfalls polymerisieren. So liegt das Umsetzungsprodukt aus Acetaldehyd und Ammoniak als Trimeres vor. Das entsprechende Umsetzungsprodukt aus Formaldehyd und Ammoniak reagiert mit weiterem Formaldehyd zu Hexamethylentetramin.

Geeignete Umsetzungsprodukte der aliphatischen Aldehyde mit Hypophosphiten und Phosphiten sind Phosphinsäuren bzw. Phosponsäuren.

Ganz besonders bevorzugte Phosphinsäuren und Phosphonssäuren sind 2-Hydroxy-2-phosphinoessigsäure und 2-Hydroxy-2-phosphonoessigsäure sowie deren Salze.

Die Einsatzmenge an aliphatischem Aldehyd oder dessen Umsetzungsprodukt mit einem aliphatischen Alkohol, einem aliphatischen Amin, Ammoniak, einem Hypophosphit oder einem Phosphit bezogen auf die wasserabsorbierenden Polymerpartikel, beträgt vorzugsweise von 0,001 bis 2 Gew.-%, besonders bevorzugt von 0,005 bis 1 Gew.-%, ganz besonders bevorzugt von 0,01 bis 0,5 Gew.-%.

Der vorliegenden Erfindung liegt die Erkenntnis zugrunde, dass aliphatische Aldehyde, d.h. Aldehyde, die keine aromatischen Ringe oder Ringsysteme aufweisen, die Verfärbungneigung wasserabsorbierender Polymerpartikel bei warmer und feuchter Lagerung deutlich vermindern. Hierbei müssen die aliphatischen Aldehyde nicht selber eingesetzt werden. Es ist auch möglich Verbindungen zu verwenden, aus denen leicht die gewünschten aliphatischen Aldehyde freigesetzt werden können. Derartige Verbindungen sind beispielsweise die Umsetzungsprodukte aliphatischer Aldehyde mit aliphatischen Alkoholen, aliphatischen Aminen, Ammoniak, Hypophosphiten oder Phosphiten.

Saccharide, Disaccharide und Polysaccharide liegen zwar auch als Halbacetale vor, sind aber für das erfindungsgemäße Verfahren weniger gut geeignet, da sie, insbesondere bei thermischer Belastung, selber zum Verfärben neigen.

Die in WO 2004/058682 A1 beschrieben Sulfinsäuren können zwar die Vergilbung unterdrücken, neigen aber zur Bildung unangenehmer Gerüche.

In einer bevorzugten Ausführungsform der vorliegenden Erfindung wird zusätzlich ein Sulfit zugesetzt. Das Sulfit kann ebenfalls vor, während oder nach einem der Schritte i) bis v) zugesetzt werden, und zwar unabhängig von der Zugabe des aliphatischen Aldehyds oder dessen Umsetzungsprodukts mit einem aliphatischen Alkohol, einem aliphatischen Amin, Ammoniak, einem Hypophosphit oder einem Phosphit.

Vorzugsweise wird das Sulfit nach Schritt iv) und vor, während oder nach Schritt v) zugesetzt. Ganz besonders bevorzugt wird der aliphatische Aldehyd oder dessen Umsetzungsprodukt mit einem aliphatischen Alkohol, einem aliphatischen Amin, Ammoniak, einem Hypophosphit oder einem Phosphit nach Schritt v) zugesetzt.

Geeignete Sulfite sind Natriumhydrogensulfit und Kaliumhydrogensulfit. Die Einsatzmenge an Sulfit, bezogen auf die wasserabsorbierenden Polymerpartikel, beträgt vorzugsweise von 0,001 bis 5 Gew.-%, besonders bevorzugt von 0,005 bis 2 Gew.-%, ganz besonders bevorzugt von 0,01 bis 1 Gew.-%.

Durch den Zusatz von Sulfiten kann die Verfärbungsneigung wasserabsorbierender Polymerpartikel weiter zurückgedrängt werden.

Im Folgenden wird die Herstellung der wasserabsorbierenden Polymerpartikel näher erläutert:

Die wasserabsorbierenden Polymerpartikel werden durch Polymerisation einer Monomerlösung oder -suspension hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraalloxyethan, Methylenbis-methacrylamid, 15-fach ethoxiliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 $g/cm^2$ durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise das Dinatriumsalz der 2-Hydroxy-2-sulfonatoessigsäure oder ein Gemisch aus dem Dinatriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; DE) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

In Verfahrensschritt i) wird die Monomerlösung oder -suspension polymerisiert. Geeignete Reaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt ii) zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Es ist aber auch möglich eine wässrige Monomerlösung zu vertropfen und die erzeugten Tropfen in einem erwärmten Trägergasstrom zu polymerisieren. Hierbei können die Verfahrensschritte Polymerisation i) und Trocknung iii) zusammengefasst werden, wie in WO 2008/040715 A2 und WO 2008/052971 A1 beschrieben.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

In Verfahrensschritt iii) wird das erhaltene Polymergel getrocknet. Die Trockner unterliegen keiner Beschränkung. Die Trocknung des Polymergels wird aber vorzugsweise mit einem Bandtrockner durchgeführt bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Moisture Content" bestimmt wird. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

In Verfahrensschritt iv) wird das getrocknete Polymergel gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 μm, besonders bevorzugt von 250 bis 600 μm, ganz besonders von 300 bis 500 μm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 μm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 μm, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 μm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur Verbesserung der Eigenschaften in einem weiteren Verfahrensschritt v) thermisch oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle A-midoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyloxazolidin-2-on, Oxazolidin-2-on und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; DE), Vrieco-Nauta Continuous Mixer (Hosokawa Micron BV; Doetinchem; NL), Processall Mixmill Mixer (Processall Incorporated; Cincinnati; US) und Schugi Flexomix® (Hosokawa Micron BV; Doetinchem; NL). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; DE), Hosokawa Bepex® Disc Dryer (Hosokawa Micron GmbH; Leingarten; DE) und Nara Paddle Dryer (NARA Machinery Europe; Frechen; DE). Überdies können auch Wirbelschichttrockner eingesetzt werden.

Die thermische Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Oberflächennachvernetzungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert.

Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Ein weiterer Gegenstand der vorliegenden Erfindung sind die gemäß dem erfindungsgemäßen Verfahren erhältlichen wasserabsorbierenden Polymerpartikel.

Ein weiterer Gegenstand der vorliegenden Erfindung sind wasserabsorbierende Polymerpartikel, enthaltend

a') mindestens ein polymerisiertes ethylenisch ungesättigtes, säuregruppentragendes Monomer a), das zumindest teilweise neutralisiert sein kann,
b') mindestens einen polymerisierten Vernetzer b),
c') optional ein oder mehrere mit den unter a) genannten Monomeren copolymerisierte ethylenisch ungesättigte Monomere d) und
d') optional ein oder mehrere wasserlösliche Polymere e),

wobei die wasserabsorbierenden Polymerpartikel mindestens einen aliphatischen Aldehyd oder dessen Umsetzungsprodukt mit einem aliphatischen Alkohol, einem aliphatischen Amin, Ammoniak, einem Hypophosphit oder einem Phosphit enthalten, wobei die oben genannten aliphatischen Aldehyde oder deren Umsetzungsprodukte mit einem aliphatischen Alkohol, einem aliphatischen Amin, Ammoniak, einem Hypophosphit oder einem Phosphit in den oben genannten Mengen eingesetzt werden können.

In einer bevorzugten Ausführungsform sind die erfindungsgemäßen Polymerpartikel mit mindestens einem aliphatischen Aldehyd oder dessen Umsetzungsprodukt mit einem aliphatischen Alkohol, einem aliphatischen Amin, Ammoniak, einem Hypophosphit oder einem Phosphit beschichtet.

Bei der Beschichtung wird der aliphatische Aldehyd oder dessen Umsetzungsprodukt mit einem aliphatischen Alkohol, einem aliphatischen Amin, Ammoniak, einem Hypophosphit oder einem Phosphit beispielsweise mit dem Polymergel nach Schritt i), vorzugsweise mit den Polymerpartikeln nach Schritt iv) und vor, während oder nach Schritt v), vermischt, wodurch in den wasserabsorbierenden Polymerpartikeln ein Konzentrationsgradient erhalten wird.

Die erfindungsgemäßen wasserabsorbierenden Polymerpartikel können zusätzlich ein Sulfit enthalten oder mit einem Sulfit beschichtet sein, wobei die oben genannten Sulfite in den oben genannten Mengen eingesetzt werden können.

Die gemäß dem erfindungsgemäßen Verfahren hergestellten wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA empfohlenen Testmethode Nr. WSP 241.2-05 "Centrifuge Retention Capacity" bestimmt.

Die gemäß dem erfindungsgemäßen Verfahren hergestellten wasserabsorbierenden Polymerpartikel weisen eine Absorption unter einem Druck von 49,2 $g/cm^2$ von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Absorption unter einem Druck von 49,2 $g/cm^2$ der wasserabsorbierenden Polymerpartikel beträgt üblicherweise weniger als 35 g/g. Die Absorption unter einem Druck von 49,2 $g/cm^2$ wird analog der von der EDANA empfohlenen Testmethode Nr. WSP 242.2-05 "Absorption under Pressure" bestimmt, wobei statt eines Drucks von 21,0 g/cm$^2$ ein Druck von 49,2 g/cm$^2$ eingestellt wird.

Ein weiterer Gegenstand der vorliegenden Erfindung sind Hygieneartikel, enthaltend erfindungsgemäße wasserabsorbierende Polymerpartikel, insbesondere Hygieneartikel zur Damenhygiene, Hygieneartikel für leichte und schwere Inkontinenz oder Kleintierstreu.

Die Hygieneartikel enthalten üblicherweise eine wasserundurchlässige Rückseite, eine wasserdurchlässige Oberseite und dazwischen einen absorbierenden Kern aus den erfindungsgemäßen wasserabsorbierenden Polymerpartikeln und Fasern, vorzugsweise Cellulose. Der Anteil der erfindungsgemäßen wasserabsorbierenden Polymerpartikel im absorbierenden Kern beträgt vorzugsweise 20 bis 100 Gew.-%, bevorzugt 50 bis 100 Gew.-%.

Die wasserabsorbierenden Polymerpartikel werden mittels der nachfolgend beschriebenen Testmethoden geprüft.

Die mit "WSP" bezeichneten Standard-Testmethoden werden beschrieben in: "Standard Test Methods for the Nonwovens Industry", Ausgabe 2005, gemeinsam herausgegeben von den "Worldwide Strategic Partners" EDANA (Avenue Eugène Plasky 157, 1030 Brussels, Belgium, www.edana.org) und INDA (1100 Crescent Green, Cary, NC 27518, U.S.A., www.inda.org). Diese Veröffentlichung ist sowohl von EDANA als auch von INDA erhältlich.

Methoden:

Die Messungen sollten, wenn nicht anders angegeben, bei einer Umgebungstemperatur von 23 $\pm$ 2 °C und einer relativen Luftfeuchte von 50 $\pm$ 10 % durchgeführt werden. Die wasserabsorbierenden Polymerpartikel werden vor der Messung gut durchmischt.

Zentrifugenretentionskapazität (Centrifuge Retention Capacity)

Die Zentrifugenretentionskapazität (CRC) wird gemäß der von der EDANA empfohlenen Testmethode Nr. WSP 241.2-05 "Centrifuge Retention Capacity" bestimmt.

Absorption unter einem Druck von 49,2 g/cm$^2$ (Absorption under Pressure)

Die Absorption unter einem Druck von 49,2 g/cm$^2$ (AUL0.7psi) wird analog der von der EDANA empfohlenen Testmethode Nr. WSP 242.2-05 "Absorption under Pressure" bestimmt, wobei statt eines Drucks von 21,0 g/cm$^2$ (AUL0.3psi) ein Druck von 49,2 g/cm$^2$ (AUL0.7psi) eingestellt wird.

Flüssigkeitsweiterleitung (Saline Flow Conductivity)

Die Flüssigkeitsweiterleitung (SFC) einer gequollenen Gelschicht unter Druckbelastung von 0,3 psi (2070 Pa) wird, wie in EP 0 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Flüssigkeitsweiterleitung (SFC) wird wie folgt berechnet:

$$\text{SFC } [cm^3s/g] = (Fg(t=0)xL0)/(dxAxWP),$$

wobei Fg(t=0) der Durchfluss an NaCl-Lösung in g/s ist, der anhand einer linearen Regressionsanalyse der Daten Fg (t) der Durchflussbestimmungen durch Extrapolation gegen t=0 erhalten wird, L0 die Dicke der Gelschicht in cm, d die Dichte der NaCl-Lösung in g/cm$^3$, A die Fläche der Gelschicht in cm$^2$ und WP der hydrostatische Druck über der Gelschicht in dyn/cm$^2$.

Gelbettpermeabilität (Gel Bed Permeability)

Die Gelbettpermeabilität (GBP) einer gequollenen Gelschicht unter Druckbelastung von 0,3 psi (2070 Pa) wird, wie in US 2005/02567575 beschrieben (Absätze [0061] und [0075]), als Gel-Bed-Permeability einer gequollenen Gelschicht aus wasserabsorbierenden Polymerpartikeln bestimmt.

CIE-Farbzahl (L, a, b)

Die Farbmessung wird entsprechend dem CIELAB-Verfahren (Hunterlab, Band 8, Jahrgang 1996, Heft 7, Seiten 1 bis 4) mit einem Colorimeter, Modell "LabScan XE S/N LX17309" (HunterLab, Reston, US) durchgeführt. Dabei werden die Farben über die Koordinaten L, a und b eines dreidimensionalen Systems beschrieben. Dabei gibt L die Helligkeit an, wobei L = 0 schwarz und L = 100 weiß bedeutet. Die Werte für a und b geben die Position der Farbe auf den Farbachsen rot/grün bzw. gelb/blau an, wobei +a für rot, -a für grün, +b für gelb und -b für blau steht. Nach der Formel HC60 = L-3b wird der HC60-Wert berechnet.

Die Farbmessung entspricht dem Dreibereichsverfahren nach DIN 5033-6.

Alterungstest

Messung 1 (anfängliche Farbe): Eine Plastikschale mit 9 cm Innendurchmesser wird mit Superabsorberpartikeln überfüllt und diese dann mit einem Messer über den Rand glatt gestrichen und die CIE-Farbzahlen sowie der HC60-Wert bestimmt.

Messung 2 (nach Alterung): Eine Plastikschale mit 9 cm Innendurchmesser wird mit Superabsorberpartikeln überfüllt und diese dann mit einem Messer über den Rand glatt gestrichen. Die Schale wird dann offen in einen auf 60 °C temperierten Klimaschrank bei konstanter relativer Luftfeuchte von 86% gestellt. Die Schale wird nach Ablauf von 21 Tagen herausgenommen. Nach Abkühlen auf Raumtemperatur werden die CIE-Farbzahlen sowie der HC60-Wert erneut bestimmt.

Beispiele

Beispiel 1:

HySorb® B 7055 (BASF SE; Ludwigshafen; DE) wurde in einem Pflugschar®-Mischer Typ M5 (Gebr. Lödige Maschinenbau GmbH; Paderborn; DE) bei 23°C und einer Wellendrehzahl von 250 Umdrehungen pro Minute mittels einer Zweistoff-Sprühdüse mit 1,0 Gew.-% einer 10 gew.-%igen Lösung von Natriumglyoxylat in entmineralisiertem Wasser beschichtet. Nach dem Aufsprühen wurde noch 15 Minuten bei einer Wellendrehzahl von 80 Umdrehungen pro Minute nachgemischt. Das erhaltene Produkt wurde auf eine Partikelgröße von weniger als 850 µm abgesiebt.

Beispiel 2:

HySorb® B 7055 (BASF SE; Ludwigshafen; DE) wurde in einem Pflugschar®-Mischer Typ M5 (Gebr. Lödige Maschinenbau GmbH; Paderborn; DE) bei 23°C und einer Wellendrehzahl von 250 Umdrehungen pro Minute mittels einer Zweistoff-Sprühdüse mit 1,0 Gew.-% einer 10 gew.-%igen Lösung von Glyoxylsäure in entmineralisiertem Wasser beschichtet. Nach dem Aufsprühen wurde noch 15 Minuten bei einer Wellendrehzahl von 80 Umdrehungen pro Minute nachgemischt. Das erhaltene Produkt wurde auf eine Partikelgröße von weniger als 850 µm abgesiebt.

Beispiel 3:

HySorb® B 7055 (BASF SE; Ludwigshafen; DE) wurde in einem Pflugschar®-Mischer Typ M5 (Gebr. Lödige Maschinenbau GmbH; Paderborn; DE) bei 23°C und einer Wellendrehzahl von 250 Umdrehungen pro Minute mittels einer Zweistoff-Sprühdüse mit 2 Gew.-% eines Gemisches aus 5 Gew.-% 2,5-Dihydroxy-1,4-dioxan, 70 Gew.-% entmineralisiertem Wasser und 25 Gew.-% Isopropanol beschichtet. Nach dem Aufsprühen wurde noch 15 Minuten bei einer Wellendrehzahl von 80 Umdrehungen pro Minute nachgemischt. Das erhaltene Produkt wurde im Vakuumtrockenschrank bei 80 °C und 250 mbar 60 Minuten lang getrocknet und auf eine Partikelgröße von weniger als 850 µm abgesiebt.

Beispiel 4:

HySorb® B 7055 (BASF SE; Ludwigshafen; DE) wurde in einem Pflugschar®-Mischer Typ M5 (Gebr. Lödige Maschinenbau GmbH; Paderborn; DE) bei 23°C und einer Wellendrehzahl von 250 Umdrehungen pro Minute mittels einer Zweistoff-Sprühdüse mit 2 Gew.-% eines Gemisches aus 5 Gew.-% 3,6-Dihydroxy-1,4-dioxan-2,5-dimethanol, 60 Gew.-% entmineralisiertem Wasser und 35 Gew.-% Ethanol beschichtet. Nach dem Aufsprühen wurde noch 15 Minuten bei einer Wellendrehzahl von 80 Umdrehungen pro Minute nachgemischt. Das erhaltene Produkt wurde im Vakuumtrockenschrank bei 80 °C und 250 mbar 60 Minuten lang getrocknet und auf eine Partikelgröße von weniger als 850 µm abgesiebt.

Beispiel 5:

HySorb® B 7055 (BASF SE; Ludwigshafen; DE) wurde in einem Pflugschar®-Mischer Typ M5 (Gebr. Lödige Maschinenbau GmbH; Paderborn; DE) bei 23°C und einer Wellendrehzahl von 250 Umdrehungen pro Minute mittels einer Zweistoff-Sprühdüse mit 1,0 Gew.-% einer 10 gew.-%igen Lösung von Hexamethylentetramin in entmineralisiertem Wasser beschichtet. Nach dem Aufsprühen wurde noch 15 Minuten bei einer Wellendrehzahl von 80 Umdrehungen pro Minute nachgemischt. Das erhaltene Produkt wurde auf eine Partikelgröße von weniger als 850 μm abgesiebt.

Die in den Beispielen 1 bis 5 beschichteten wasserabsorbierenden Polymerpartikel wurden dem Alterungstest unterzogen. Die Ergebnisse sind in Tabelle 1 zusammengestellt. Die Beispiele zeigen, dass die erfindungsgemäßen wasserabsorbierenden Polymerpartikel nach Alterung deutlich heller und weniger verfärbt sind.

Die in den Beispielen 1 bis 5 eingesetzten wasserabsorbierenden Polymerpartikel vom Typ HySorb® B 7055 hatten eine CIE-Farbzahl von L = 93,4, a = 3,7 und b = 4,8, sowie einen HC60-Wert von 79,1. HySorb® B 7055 sind handelsübliche oberflächennachvernetzte wasserabsorbierende Polymerpartikel.

### Tab. 1: Zusatz nach der Oberflächenachvernetzung

|  | L | a | b | HC 60 |
|---|---|---|---|---|
| HySorb® B 7055 | 64,8 | 4,8 | 17,8 | 11,5 |
| Bsp. 1 | 82,1 | 1,2 | 11,1 | 48,8 |
| Bsp. 2 | 82,6 | 0,9 | 10,8 | 50,2 |
| Bsp. 3 | 79,6 | 1,4 | 11,6 | 44,8 |
| Bsp. 4 | 80,2 | 1,3 | 11,9 | 44,5 |
| Bsp. 5 | 78,4 | 1,7 | 12,1 | 42,1 |

Beispiel 6: Herstellung wasserabsorbierender Polymerpartikel

In einem 2 l Edelstahlbecher wurden 326,7 g 50 Gew.-%ige Natronlauge und 675 g gefrorenes, entmineralisiertes Wasser vorgelegt. Unter Rühren wurden 392,0 g Acrylsäure zugegeben, wobei die die Geschwindigkeit der Zugabe so eingestellt wurde, dass die Temperatur 35°C nicht überstieg. Der Mischung wurde dann unter Rühren mit Hilfe eines Kühlbades abgekühlt. Als die Temperatur der Mischung auf 20°C abgefallen war, wurden 1,08 g dreifach ethoxyliertes Glycerintriacrylat, 0,041 g 2-Hydroxy-2-methyl-1-phenylpropan-1-on (DAROCUR®1173; Ciba Specialty Chemicals Inc.; Basel; CH) und 0,014 g 2,2-Dimethoxy-1,2-diphenylethan-1-on (IRGACURE® 651; Ciba Specialty Chemicals Inc.; Basel; CH) zugesetzt. Es wurde weiter abgekühlt, und bei Erreichen von 15°C wurde die Mischung durch Durchleiten von Stickstoff mittels einer Glasfritte von Sauerstoff befreit. Beim Erreichen von 0°C wurden 0,51 g Natriumpersulfat (gelöst in 5 ml entmineralisiertem Wasser) und 0,2 g 30 gew.-%ige Wasserstoffperoxidlösung (gelöst in 6 ml entmineralisiertem Wasser) zugesetzt und die Monomerlösung wurde in eine Glasschale überführt. Die Glasschale war so dimensioniert, dass sich eine Schichtdicke der Monomerlösung von 5 cm einstellte. Anschließend wurden 0,047 g Gemisch aus dem Natriumsalz der 2-Hydroxy-2-sulfinatoessigsäure, dem Dinatriumsalz der 2-Hydroxy-2-sulfonatoessigsäure und Natriumbisulfit (Brüggolite® FF6; Brüggemann Chemicals,; Heilbronn; DE), gelöst in 5 ml entmineralisiertem Wasser, zugesetzt und die Monomerlösung mit Hilfe eines Glasstabes kurz durchgerührt. Die Glasschale mit der Monomerlösung wurde unter eine UV-Lampe gestellt (UV-Intensität = 25 mW/cm$^2$), wobei die Polymerisation einsetzte. Nach 16 Minuten wurde das erhaltene Polymergel dreimal mit Hilfe eines handelsüblichen Fleischwolfs mit 6 mm Lochscheibe extrudiert und im Umlufttrockenschrank bei 160°C eine Stunde lang getrocknet. Das getrocknete Polymergel wurde dann gemahlen und auf eine Partikelgröße von 150 bis 850 μm abgesiebt.

Dieses Grundpolymer wurde zur Oberflächennachvernetzung in einem Pflugschar®-Mischer Typ M5 mit Heizmantel (Gebr. Lödige Maschinenbau GmbH; Paderborn; DE) bei 23°C und einer Wellendrehzahl von 450 Umdrehungen pro Minute mittels einer Zweistoff-Sprühdüse mit einem Gemisch aus 0,10 Gew.-% Ethylenglykoldiglycidylether (Denacol® EX-810; Nagase ChemteX Corporation; Osaka; JP), 1,50 Gew.-% 1,2-Propandiol, 2,8 Gew.-% entmineralisiertem Wasser und 0,4 Gew.-% wässriger Aluminiumsulfat-Lösung (26,8 gew.-%ig), jeweils bezogen auf das Grundpolymer, beschichtet.

Nach dem Aufsprühen wurde die Produkttemperatur auf 150°C erhöht und das Reaktionsgemisch 60 Minuten lang bei dieser Temperatur und einer Wellendrehzahl von 80 Umdrehungen pro Minute gehalten. Das erhaltene Produkt wurde wieder auf Umgebungstemperatur abkühlen gelassen und gesiebt. Die oberflächennachvernetzten wasserabsorbierenden Polymerpartikel wurden auf eine Partikelgröße von 150 μm bis 850 μm abgesiebt und wiesen folgende Eigenschaften auf:

CRC = 31,6 g/g

(fortgesetzt)

| AUL0.7psi | = 22,9 g/g |
|---|---|
| SFC | = 25 x $10^{-7}$ cm$^3$s/g |
| GBP | = 15 Darcies |

Die erhaltenen wasserabsorbierenden Polymerpartikel hatten eine CIE-Farbzahl von L = 88,7, a = -0,4 und b = 9,0, sowie einen HC60-Wert von 61,7.

Beispiel 7:

Es wurde verfahren wie unter Beispiel 6. Vor dem Extrudieren des Polymergels wurden 0,20 g Glyoxylsäure zugesetzt.

Beispiel 8

Es wurde verfahren wie unter Beispiel 6. Vor dem Extrudieren des Polymergels wurden 0,30 g Kaliumglyoxylat zugesetzt.

Beispiel 9

Es wurde verfahren wie unter Beispiel 6. Vor dem Extrudieren des Polymergels wurden 1,2 g Glyzerinaldehyd zugesetzt.

Die in den Beispielen 6 bis 9 hergestellten wasserabsorbierenden Polymerpartikel wurden dem Alterungstest unterzogen. Die Ergebnisse sind in Tabelle 2 zusammengestellt. Die Beispiele zeigen, dass bereits geringe Mengen des Aldehyds die erfindungsgemäßen wasserabsorbierenden Polymerpartikel gegen Verfärbung bei Alterung stabilisieren.

Tab. 2: Zusatz nach der Polymerisation

| | L | a | b | HC 60 |
|---|---|---|---|---|
| Bsp. 6 (Vgl.) | 68,1 | 3,0 | 13,2 | 28,5 |
| Bsp. 7 | 81,5 | 1,4 | 11,2 | 47,9 |
| Bsp. 8 | 82,4 | 0,8 | 10,4 | 51,2 |
| Bsp. 9 | 79,7 | 1,3 | 11,0 | 46,7 |

Beispiel 10: Herstellung wasserabsorbierender Polymerpartikel

14,3 kg wässrige Natriumacrylat-Lösung (37,5 gew.-%ig), 1,4 kg Acrylsäure und 350 g entmineralisiertes Wasser wurden mit 8,5 g dreifach ethoxyliertem Glycerintriacrylat gemischt. Diese Lösung wurde in einem erwärmten, mit Stickstoffatmosphäre gefüllten Vertropfungsturm (180°C, 12 m Höhe, 2 m Durchmesser, Gasgeschwindigkeit 0,1 m/s im Gleichstrom, Vertropfer mit 40 mm Durchmesser, 2 mm Innenhöhe und einer Vertropferplatte mit 60 Bohrungen von je 200 $\mu$m Durchmesser) in einer Dosiergeschwindigkeit von 32 kg/h vertropft. Die Temperatur der Lösung betrug 25°C. Kurz vor dem Vertropfer wurde die Monomerlösung über einen statischen Mischer mit zwei Initiatorlösungen gemischt. Als Initiator 1 wurde eine 3 gew.-%ige Lösung von 2,2'-Azobis-[2-(2-imidazolin-2-yl)-propan]-dihydrochlorid in entmineralisiertem Wasser und als Initiator 2 eine 6,1 gew.-%ige Lösung von Natriumperoxodisulfat in entmineralisiertem Wasser verwendet. Die Dosiergeschwindigkeit der Initiatorlösung 1 betrug 0,932 kg/h und die Dosiergeschwindigkeit der Initiatorlösung 2 betrug 0,629 kg/h. Die erhaltenen Polymerpartikel wurden auf eine Partikelgröße von 150 bis 850 $\mu$m abgesiebt, um eventuell gebildete Agglomerate abzutrennen, und wiesen folgende Eigenschaften auf:

| CRC | = 30,4 g/g |
|---|---|
| AUL0.7psi | = 22,9 g/g |
| SFC | = 24 x $10^{-7}$ cm$^3$s/g |
| GBP | = 8 Darcies |

Die erhaltenen wasserabsorbierenden Polymerpartikel hatten eine CIE-Farbzahl von L = 93,1, a = 0,5 und b = 3,2, sowie einen HC60-Wert von 83,5.

Beispiel 11: Herstellung wasserabsorbierender Polymerpartikel

14,3 kg wässrige Natriumacrylat-Lösung (37,5 gew.-%ig), 1,4 kg Acrylsäure und 350 g entmineralisiertes Wasser wurden mit 8,5 g dreifach ethoxyliertem Glycerintriacrylat gemischt. Diese Lösung wurde in einem erwärmten, mit Stickstoffatmosphäre gefüllten Vertropfungsturm (180°C, 12 m Höhe, 2 m Durchmesser, Gasgeschwindigkeit 0,1 m/s im Gleichstrom, Vertropfer mit 40 mm Durchmesser, 2 mm Innenhöhe und einer Vertropferplatte mit 60 Bohrungen von je 200 $\mu$m Durchmesser) in einer Dosiergeschwindigkeit von 32 kg/h vertropft. Die Temperatur der Lösung betrug 25°C. Kurz vor dem Vertropfer wurde die Monomerlösung über einen statischen Mischer mit drei Initiatorlösungen gemischt. Als Initiator 1 wurden eine 3 gew.-%ige Lösung von 2,2'-Azobis-[2-(2-imidazolin-2-yl)-propan]-dihydrochlorid in entmineralisiertem Wasser, als Initiator 2 eine 6,1 gew.-%ige Lösung von Natriumperoxodisulfat in entmineralisiertem Wasser und als Initiator 3 wurde eine 3 gew.-%ige Lösung von dem Dinatriumsalz der 2-Hydroxy-2-sulfonatoessigsäure in entionisiertem Wasser verwendet. Die Dosiergeschwindigkeit der Initiatorlösung 1 betrug 0,548 kg/h, die Dosiergeschwindigkeit der Lösung 2 betrug 0,449 kg/h und die Dosiergeschwindigkeit der Lösung 3 betrug 0,183 kg/h. Die erhaltenen Polymerpartikel wurden auf eine Partikelgröße von 150 bis 850 $\mu$m abgesiebt, um eventuell gebildete Agglomerate abzutrennen, und wiesen folgende Eigenschaften auf:

| | |
|---|---|
| CRC | = 31,8 g/g |
| AUL0.7psi | = 22,5 g/g |
| SFC | = 19 x $10^{-7}$ cm$^3$s/g |
| GBP | = 7 Darcies |

Die erhaltenen wasserabsorbierenden Polymerpartikel hatten eine CIE-Farbzahl von L = 93,6, a = 0,4 und b = 2,7, sowie einen HC60-Wert von 85,5.

Beispiel 12:

1000 g der wasserabsorbierenden Polymerpartikel von Beispiel 10 wurden in einem Pflugschar®-Mischer Typ M5 (Gebr. Lödige Maschinenbau GmbH; Paderborn; DE) bei 23°C und einer Wellendrehzahl von 250 Umdrehungen pro Minute mittels zwei Zweistoff-Sprühdüsen gleichzeitig mit einer Lösung von 1,5 g Natriumglyoxylat in 15 g entmineralisiertem Wasser und mit einer Lösung von 1,9 g Natriumbisulfit in 15 g entmineralisiertem Wasser beschichtet. Nach dem Aufsprühen wurde noch 15 Minuten bei einer Wellendrehzahl von 80 Umdrehungen pro Minute nachgemischt. Das erhaltene Produkt wurde auf eine Partikelgröße von weniger als 850 $\mu$m abgesiebt.

Beispiel 13

1000 g der wasserabsorbierenden Polymerpartikel von Beispiel 10 wurden in einem Pflugschar®-Mischer Typ M5 (Gebr. Lödige Maschinenbau GmbH; Paderborn; DE) bei 23°C und einer Wellendrehzahl von 250 Umdrehungen pro Minute mittels zwei Zweistoff-Sprühdüsen gleichzeitig mit einer Lösung von 1,5 g Natriumglyoxylat in 15 g entmineralisiertem Wasser und mit einer Lösung von 4,5 g Natriumbisulfit in 20 g entmineralisiertem Wasser beschichtet. Nach dem Aufsprühen wurde noch 15 Minuten bei einer Wellendrehzahl von 80 Umdrehungen pro Minute nachgemischt. Das erhaltene Produkt wurde auf eine Partikelgröße von weniger als 850 $\mu$m abgesiebt.

Beispiel 14

1000 g der wasserabsorbierenden Polymerpartikel von Beispiel 10 wurden in einem Pflugschar®-Mischer Typ M5 (Gebr. Lödige Maschinenbau GmbH; Paderborn; DE) bei 23°C und einer Wellendrehzahl von 250 Umdrehungen pro Minute mittels zwei Zweistoff-Sprühdüsen gleichzeitig mit einer Lösung von 1,5 g Natriumglyoxylat in 15 g entmineralisiertem Wasser und mit einer Lösung von 0,8 g Natriumbisulfit in 10 g entmineralisiertem Wasser beschichtet. Nach dem Aufsprühen wurde noch 15 Minuten bei einer Wellendrehzahl von 80 Umdrehungen pro Minute nachgemischt. Das erhaltene Produkt wurde auf eine Partikelgröße von weniger als 850 $\mu$m abgesiebt.

Beispiel 15

1000 g der wasserabsorbierenden Polymerpartikel von Beispiel 11 wurden in einem Pflugschar®-Mischer Typ M5 (Gebr. Lödige Maschinenbau GmbH; Paderborn; DE) bei 23°C und einer Wellendrehzahl von 80 Umdrehungen pro Minute mit 1,5 g mit einer hydrophoben Fällungskieselsäure (Typ Sipernat® D-17; Evonik Degussa GmbH; Frankfurt am Main; DE) versetzt. Nach 5-minütiger Mischzeit wurde die Wellendrehzahl auf 250 Umdrehungen pro Minute erhöht und mittels einer Zweistoff-Sprühdüse eine Lösung von 0,4 g Glyoxylsäure in 20 g entmineralisiertem Wasser aufgebracht. Nach dem Aufsprühen wurde noch 15 Minuten bei einer Wellendrehzahl von 80 Umdrehungen pro Minute nachgemischt. Das erhaltene Produkt wurde auf eine Partikelgröße von weniger als 850 μm abgesiebt.

Die in den Beispielen 10 bis 15 hergestellten wasserabsorbierenden Polymerpartikel wurden dem Alterungstest unterzogen. Die Ergebnisse sind in Tabelle 3 zusammengestellt. Die Beispiele zeigen, dass die erfindungsgemäßen wasserabsorbierenden Polymerpartikel nach Alterung deutlich heller und weniger verfärbt sind.

Tab. 3: Zusatz nach der Trocknung

|  | L | a | b | HC60 |
|---|---|---|---|---|
| Bsp. 10 (Vgl.) | 70,4 | 3,9 | 18,5 | 14,9 |
| Bsp. 11 (Vgl.) | 75,2 | 2,8 | 14,0 | 33,2 |
| Bsp. 12 | 79,6 | 1,2 | 11,9 | 43,9 |
| Bsp. 13 | 80,2 | 1,4 | 11,6 | 45,4 |
| Bsp. 14 | 80,0 | 1,3 | 11,9 | 44,3 |
| Bsp. 15 | 85,7 | 0,4 | 8,7 | 59,6 |

Beispiel 16: Herstellung wasserabsorbierender Polymerpartikel

In einen Pflugschar®-Schaufeltrockner Typ VT 5R-MK mit 5 l Volumen und Heiz-/Kühlmantel (Gebr. Lödige Maschinenbau GmbH; Paderborn: DE) wurden 459 g entmineralisiertes Wasser, 213,9 g Acrylsäure, 1924,9 g wässrige Natriumacrylat-Lösung (37,3 gew.-%ig) und 2,52 g dreifach ethoxyliertes Glycerintriacrylat, vorgelegt und unter Durchperlen von Stickstoff 20 Minuten inertisiert. Die Welle des Reaktors wurde durchgehend mit 96 Umdrehungen pro Minute gedreht. Die Reaktionsmischung wurde dabei von außen so gekühlt, dass die nachfolgende Initiatorzugabe bei ca. 20°C erfolgte. Schließlich wurden in den Pflugschar®-Schaufeltrockner unter Rühren noch 2,139 g Natriumpersulfat (gelöst in 12,12 g entmineralisiertem Wasser), 0,046 g Ascorbinsäure (gelöst in 9,12 g entmineralisiertem Wasser) und 0,127 g 30 gew.-%ige wässrige Wasserstoffperoxidlösung (verdünnt mit 1,15 g entmineralisiertem Wasser) rasch hintereinander hinzugefügt. Die Reaktion setzte zügig ein und bei Erreichen einer Innentemperatur von 30°C wurde der Mantel mit 80°C heißem Wärmeträgermedium beheizt, um die Reaktion möglichst adiabat zu Ende zu führen. Nach Erreichen der Maximaltemperatur wurde wiederum gekühlt (Kühlflüssigkeit mit -12°C), so das entstandene Polymergel auf unter 50°C herabgekühlt und dann ausgetragen wurde. Das Polymergel wurde dann im Umlufttrockenschrank bei 160°C eine Stunde lang getrocknet. Das getrocknete Polymergel wurde dann gemahlen und auf eine Partikelgröße von 150 bis 710 μm abgesiebt.

Dieses Grundpolymer wurde zur Oberflächennachvernetzung in einem Pflugschar®-Mischer Typ M5 mit Heizmantel (Gebr. Lödige Maschinenbau GmbH; Paderborn; DE) bei 23°C und einer Wellendrehzahl von 450 Umdrehungen pro Minute mittels einer Zweistoff-Sprühdüse mit einem Gemisch aus 0,125 Gew.-% N-(2-Hydroxyethyl)-2-oxazolidinon, 1,5 Gew.-% entmineralisiertem Wasser, 1,5 Gew.% 1,3-Propandiol, 0,003 Gew.-% Sorbitanmonococoat (Span® 20) und 2,8 Gew.-% wässriger Aluminium-laktat-Lösung (25 gew.-%ig), jeweils bezogen auf das Grundpolymer, beschichtet. Nach dem Aufsprühen wurde die Produkttemperatur auf 175°C erhöht und das Reaktionsgemisch 80 Minuten lang bei dieser Temperatur und einer Wellendrehzahl von 80 Umdrehungen pro Minute gehalten. Das erhaltene Produkt wurde wieder auf Umgebungstemperatur abkühlen gelassen und gesiebt. Die oberflächennachvernetzten wasserabsorbierenden Polymerpartikel wurden auf eine Partikelgröße von 150 bis 710 μm abgesiebt.

Die erhaltenen wasserabsorbierenden Polymerpartikel hatten eine CIE-Farbzahl von L = 92,2, a = -0,4 und b = 6,5, sowie einen HC60-Wert von 72,2.

Beispiel 17:

Es wurde verfahren wie unter Beispiel 16. Vor der Polymerisation wurden 1,2 g Natrium-2,2-dimethoxyacetat im Pflugschar®-Schaufeltrockner vorgelegt.

Beispiel 18:

Es wurde verfahren wie unter Beispiel 16. Das Grundpolymer wurde zusätzlich mit 0,10 Gew.-% Glyoxylsäure, bezogen auf Grundpolymer, beschichtet.

Beispiel 19

Es wurde verfahren wie unter Beispiel 16. Das Grundpolymer wurde zusätzlich mit 0,15 Gew.-% Natriumglyoxylat, bezogen auf Grundpolymer, beschichtet.

Beispiel 20

Das in Beispiel 16 hergestellte Polymer wurde in einem Pflugschar®-Mischer Typ M5 (Gebr. Lödige Maschinenbau GmbH; Paderborn; DE) bei 23°C und einer Wellendrehzahl von 250 Umdrehungen pro Minute mittels einer Zweistoff-Sprühdüse mit 1,5 Gew.-% einer 10 gew.-%igen Lösung von 2-Hydroxy-2-phosphonoessigsäure in entmineralisiertem Wasser beschichtet. Nach dem Aufsprühen wurde noch 15 Minuten bei einer Wellendrehzahl von 80 Umdrehungen pro Minute nachgemischt. Das erhaltene Produkt wurde auf eine Partikelgröße von weniger als 850 $\mu$m abgesiebt.

Die in den Beispielen 16 bis 20 hergestellten wasserabsorbierenden Polymerpartikel wurden dem Alterungstest unterzogen. Die Ergebnisse sind in Tabelle 4 und 5 zusammengestellt. Die Beispiele zeigen, dass die erfindungsgemäßen wasserabsorbierenden Polymerpartikel nach Alterung deutlich heller und weniger verfärbt sind, ohne dass die Absorptionseigenschaften beeinträchtigt wären.

Tab. 4: Absorptionseigenschaften

|  | CRC [g/g] | AUL0.7psi [g/g] | SFC [10-7 cm$^3$s/g] | GBP [Darcies] |
|---|---|---|---|---|
| Bsp. 16 (Vgl.) | 28,7 | 23,9 | 125 | 15 |
| Bsp. 17 | 29,2 | 23,3 | 110 | 14 |
| Bsp. 18 | 28,9 | 23,5 | 123 | 16 |
| Bsp. 19 | 28,5 | 24,1 | 129 | 14 |
| Bsp. 20 | 28,4 | 23,8 | 116 | 17 |

Tab. 5: Zusatz vor der Polymerisation bzw. vor oder nach der thermischen Oberflächennachvernetzung

|  | L | a | b | HC60 |
|---|---|---|---|---|
| Bsp. 16 (Vgl.) | 65,1 | 4,9 | 17,3 | 13,2 |
| Bsp. 17 | 79,7 | 2,1 | 12,1 | 43,4 |
| Bsp. 18 | 81,4 | 1,0 | 11,4 | 47,2 |
| Bsp. 19 | 82,3 | 0,9 | 10,2 | 51,7 |
| Bsp. 20 | 82,0 | 1,2 | 10,8 | 49,6 |

**Patentansprüche**

1. Verfahren zur Herstellung wasserabsorbierender Polymerpartikel durch Polymerisation einer Monomerlösung oder -suspension, enthaltend

   a) mindestens ein ethylenisch ungesättigtes, säuregruppentragendes Monomer, das zumindest teilweise neutralisiert sein kann,
   b) mindestens einen Vernetzer,
   c) mindestens einen Initiator,
   d) optional ein oder mehrere mit den unter a) genannten Monomeren copolymerisierbare ethylenisch ungesättigte Monomere und
   e) optional ein oder mehrere wasserlösliche Polymere,
   umfassend die Schritte Polymerisation der Monomerlösung zu einem Polymergel i), optional Zerkleinerung des erhaltenen Polymergels ii), Trocknung des Polymergels iii), Mahlung und Klassierung des getrockneten Polymergels zu Polymerpartikeln iv), und optional thermischer Oberflächennachvernetzung der klassierten Polymerpartikel v), **dadurch gekennzeichnet, dass** vor, während oder nach einem der Schritte i) bis v) mindestens ein aliphatischer Aldehyd oder dessen Umsetzungsprodukt mit einem aliphatischen Alkohol, einem aliphatischen Amin, Ammoniak, einem Hypophosphit oder einem Phosphit zugesetzt wird.

2. Verfahren gemäß Anspruch 1, **dadurch gekennzeichnet, dass** der aliphatische Aldehyd oder dessen Umsetzungsprodukt mit einem aliphatischen Alkohol, einem aliphatischen Amin, Ammoniak, einem Hypophosphit oder einem Phosphit nach Schritt iv) und vor, während oder nach Schritt v) zugesetzt wird.

3. Verfahren gemäß Anspruch 1 oder 2, **dadurch gekennzeichnet, dass** aliphatische Aldehyd oder dessen Umsetzungsprodukt mit einem aliphatischen Alkohol, einem aliphatischen Amin, Ammoniak, einem Hypophosphit oder einem Phosphit nach Schritt v) zugesetzt wird.

4. Verfahren gemäß einem der Ansprüche 1 bis 3, **dadurch gekennzeichnet, dass** aliphatische Aldehyd oder dessen Umsetzungsprodukt mit einem aliphatischen Alkohol, einem aliphatischen Amin, Ammoniak, einem Hypophosphit oder einem Phosphit ausgewählt ist aus der Gruppe bestehend aus Glyoxylsäure, Natriumglyoxylat, Kaliumglyoxylat, Glyzerinaldehyd, 2,5-Dihydroxy-1,4-dioxan, 3,6-Dihydroxy-2,5-dihydroxymethyl-1,4-dioxan, Natrium-2,2-dimethoxyacetat, Hexamethylentetramin und 2-Hydroxy-2-phosphonoessigsäure.

5. Verfahren gemäß einem der Ansprüche 1 bis 4, **dadurch gekennzeichnet, dass** der aliphatische Aldehyd oder dessen Umsetzungsprodukt mit einem aliphatischen Alkohol, einem aliphatischen Amin, Ammoniak, einem Hypophosphit oder einem Phosphit ausgewählt ist aus der Gruppe bestehend aus Glyoxylsäure, Natriumglyoxylat und Kaliumglyoxylat.

6. Verfahren gemäß einem der Ansprüche 1 bis Anspruch 5, **dadurch gekennzeichnet, dass** von 0,01 bis 0,5 Gew.-% aliphatischer Aldehyd oder dessen Umsetzungsprodukt mit einem aliphatischen Alkohol, einem aliphatischen Amin, Ammoniak, einem Hypophosphit oder einem Phosphit, bezogen auf die wasserabsorbierenden Polymerpartikel, zugesetzt wird.

7. Verfahren gemäß einem der Ansprüche 1 bis 6, **dadurch gekennzeichnet, dass** das zusätzlich ein Sulfit zugesetzt wird.

8. Verfahren gemäß Anspruch 7, **dadurch gekennzeichnet, dass** das Sulfit ausgewählt ist aus der Gruppe bestehend aus Natriumhydrogensulfit und Kaliumhydrogensulfit.

9. Verfahren gemäß Anspruch 7 oder 8, **dadurch gekennzeichnet, dass** von 0,01 bis 1 Gew.-% Sulfit, bezogen auf die wasserabsorbierenden Polymerpartikel, zugesetzt wird.

10. Wasserabsorbierende Polymerpartikel, erhältlich nach einem Verfahren gemäß einem der Ansprüche 1 bis 9.

11. Wasserabsorbierende Polymerpartikel, enthaltend

a') mindestens ein polymerisiertes ethylenisch ungesättigtes, säuregruppentragendes Monomer, das zumindest teilweise neutralisiert sein kann,
b') mindestens einen polymerisierten Vernetzer,
c') optional ein oder mehrere mit den unter a') genannten Monomeren copolymerisierte ethylenisch ungesättigte Monomere und
d') optional ein oder mehrere wasserlösliche Polymere,
wobei die wasserabsorbierenden Polymerpartikel mindestens einen aliphatischen Aldehyd oder dessen Umsetzungsprodukt mit einem aliphatischen Alkohol, einem aliphatischen Amin, Ammoniak, einem Hypophosphit oder einem Phosphit enthalten.

12. Polymerpartikel gemäß Anspruch 11, wobei die wasserabsorbierenden Polymerpartikel mit mindestens einem aliphatischen Aldehyd oder dessen Umsetzungsprodukt mit einem aliphatischen Alkohol, einem aliphatischen Amin, Ammoniak, einem Hypophosphit oder einem Phosphit beschichtet sind.

13. Polymerpartikel gemäß Anspruch 11 oder 12, wobei die wasserabsorbierenden Polymerpartikel zusätzlich ein Sulfit enthalten oder mit einem Sulfit beschichtet sind.

14. Polymerpartikel gemäß einem der Ansprüche 10 bis 13, wobei die wasserabsorbierenden Polymerpartikel eine Zentrifugenretentionskapazität von mindestens 15 g/g aufweisen.

15. Hygieneartikel, enthaltend wasserabsorbierende Polymerpartikel gemäß einem der Ansprüche 10 bis 14.

**Claims**

1. A process for producing water-absorbing polymer particles by polymerizing a monomer solution or suspension comprising

   a) at least one ethylenically unsaturated monomer which bears acid groups and may be at least partly neutralized,
   b) at least one crosslinker,
   c) at least one initiator,
   d) optionally one or more ethylenically unsaturated monomers copolymerizable with the monomers mentioned under a) and
   e) optionally one or more water-soluble polymers,
   comprising the steps of polymerizing the monomer solution to give a polymer gel i), optionally comminuting the resulting polymer gel ii), drying the polymer gel iii), grinding and classifying the dried polymer gel to polymer particles iv), and optionally thermally surface postcrosslinking the classified polymer particles v), which comprises adding at least one aliphatic aldehyde or reaction product thereof with an aliphatic alcohol, an aliphatic amine, ammonia, a hypophosphite or a phosphite before, during or after one of steps i) to v).

2. The process according to claim 1, wherein the aliphatic aldehyde or reaction product thereof with an aliphatic alcohol, an aliphatic amine, ammonia, a hypophosphite or a phosphite is added after step iv) and before, during or after step v).

3. The process according to claim 1 or 2, wherein the aliphatic aldehyde or reaction product thereof with an aliphatic alcohol, an aliphatic amine, ammonia, a hypophosphite or a phosphite is added after step v).

4. The process according to any of claims 1 to 3, wherein the aliphatic aldehyde or reaction product thereof with an aliphatic alcohol, an aliphatic amine, ammonia, a hypophosphite or a phosphite is selected from the group consisting of glyoxylic acid, sodium glyoxylate, potassium glyoxylate, glyceraldehyde, 2,5-dihydroxy-1,4-dioxane, 3,6-dihydroxy-2,5-dihydroxymethyl-1,4-dioxane, sodium 2,2-dimethoxyacetate, hexamethylenetetramine and 2-hydroxy-2-phosphonoacetic acid.

5. The process according to any of claims 1 to 4, wherein the aliphatic aldehyde or reaction product thereof with an aliphatic alcohol, an aliphatic amine, ammonia, a hypophosphite or a phosphite is selected from the group consisting of glyoxylic acid, sodium glyoxylate and potassium glyoxylate.

6. The process according to any of claims 1 to 5, wherein from 0.01 to 0.5% by weight of aliphatic aldehyde or reaction product thereof with an aliphatic alcohol, an aliphatic amine, ammonia, a hypophosphite or a phosphite, based on the water-absorbing polymer particles, is added.

7. The process according to any of claims 1 to 6, wherein a sulfite is additionally added.

8. The process according to claim 7, wherein the sulfite is selected from the group consisting of sodium hydrogensulfite and potassium hydrogensulfite.

9. The process according to claim 7 or 8, wherein from 0.01 to 1% by weight of sulfite, based on the water-absorbing polymer particles, is added.

10. Water-absorbing polymer particles obtainable by a process according to any of claims 1 to 9.

11. Water-absorbing polymer particles comprising

    a') at least one polymerized ethylenically unsaturated monomer which bears acid groups and may be at least partly neutralized,
    b') at least one polymerized crosslinker,
    c') optionally one or more ethylenically unsaturated monomers copolymerized with the monomers mentioned under a') and
    d') optionally one or more water-soluble polymers,
    said water-absorbing polymer particles comprising at least one aliphatic aldehyde or reaction product thereof with an aliphatic alcohol, an aliphatic amine, ammonia, a hypophosphite or a phosphite.

**12.** Polymer particles according to claim 11, which have been coated with at least one aliphatic aldehyde or reaction product thereof with an aliphatic alcohol, an aliphatic amine, ammonia, a hypophosphite or a phosphite.

**13.** Polymer particles according to claim 11 or 12, which additionally comprise a sulfite or have been coated with a sulfite.

**14.** Polymer particles according to any of claims 10 to 13, which have a centrifuge retention capacity of at least 15 g/g.

**15.** A hygiene article comprising water-absorbing polymer particles according to any of claims 10 to 14.

**Revendications**

**1.** Procédé pour la préparation de particules polymères absorbant l'eau par polymérisation d'une solution ou d'une suspension de monomères, contenant

a) au moins un monomère éthyléniquement insaturé, portant des groupes acides, qui peut être au moins partiellement neutralisé,
b) au moins un agent de réticulation,
c) au moins un initiateur,
d) éventuellement un ou plusieurs monomères éthyléniquement insaturés copolymérisables avec les monomères cités en a) et
e) éventuellement un ou plusieurs polymères solubles dans l'eau,
comprenant les étapes de polymérisation de la solution de monomères en un gel polymère i), éventuellement broyage du gel polymère obtenu ii), séchage du gel polymère iii), broyage et classification du gel polymère séché en particules polymères iv) et éventuellement post-réticulation superficielle thermique des particules polymères classées v), **caractérisé en ce qu'**on ajoute, avant, pendant ou après une des étapes i) à v) au moins un aldéhyde aliphatique ou son produit de transformation avec un alcool aliphatique, une amine aliphatique, de l'ammoniac, un hypophosphite ou un phosphite.

**2.** Procédé selon la revendication 1, **caractérisé en ce que** l'aldéhyde aliphatique ou son produit de transformation avec un alcool aliphatique, une amine aliphatique, de l'ammoniac, un hypophosphite ou un phosphite est ajouté après l'étape iv) et avant, pendant ou après l'étape v).

**3.** Procédé selon la revendication 1 ou 2, **caractérisé en ce que** l'aldéhyde aliphatique ou son produit de transformation avec un alcool aliphatique, une amine aliphatique, de l'ammoniac, un hypophosphite ou un phosphite est ajouté après l'étape v).

**4.** Procédé selon l'une quelconque des revendications 1 à 3, **caractérisé en ce que** l'aldéhyde aliphatique ou son produit de transformation avec un alcool aliphatique, une amine aliphatique, de l'ammoniac, un hypophosphite ou un phosphite est choisi dans le groupe constitué par l'acide glyoxylique, le glyoxylate de sodium, le glyoxylate de potassium, l'aldéhyde glycérique, le 2,5-dihydroxy-1,4-dioxane, le 3,6-dihydroxy-2,5-dihydroxyméthyl-1,4-dioxane, le 2,2-diméthoxyacétate de sodium, l'hexaméthylènetétraamine et l'acide 2-hydroxy-2-phosphonoacétique.

**5.** Procédé selon l'une quelconque des revendications 1 à 4, **caractérisé en ce que** l'aldéhyde aliphatique ou son produit de transformation avec un alcool aliphatique, une amine aliphatique, de l'ammoniac, un hypophosphite ou un phosphite est choisi dans le groupe constitué par l'acide glyoxylique, le glyoxylate de sodium et le glyoxylate de potassium.

**6.** Procédé selon l'une quelconque des revendications 1 à la revendication 5, **caractérisé en ce qu'**on ajoute 0,01 à 0,5% en poids d'aldéhyde aliphatique ou son produit de transformation avec un alcool aliphatique, une amine aliphatique, de l'ammoniac, un hypophosphite ou un phosphite, par rapport aux particules polymères absorbant l'eau.

**7.** Procédé selon l'une quelconque des revendications 1 à 6, **caractérisé en ce qu'**on ajoute en plus un sulfite.

**8.** Procédé selon la revendication 7, **caractérisé en ce que** le sulfite est choisi dans le groupe constitué par l'hydrogénosulfite de sodium et l'hydrogénosulfite de potassium.

**9.** Procédé selon la revendication 7 ou 8, **caractérisé en ce qu'**on ajoute 0,01 à 1% en poids de sulfite, par rapport aux particules polymères absorbant l'eau.

**10.** Particules polymères absorbant l'eau, pouvant être obtenues selon un procédé selon l'une quelconque des revendications 1 à 9.

**11.** Particules polymères absorbant l'eau, contenant

a') au moins un monomère éthyléniquement insaturé polymérisé, portant des groupes acides, qui peut être au moins partiellement neutralisé,

b') au moins un réticulant polymérisé,

c') éventuellement un ou plusieurs monomères éthyléniquement insaturés copolymérisés avec les monomères cités en a') et

d') éventuellement un ou plusieurs polymères solubles dans l'eau,

les particules polymères absorbant l'eau contenant au moins un aldéhyde aliphatique ou son produit de transformation avec un alcool aliphatique, une amine aliphatique, de l'ammoniac, un hypophosphite ou un phosphite.

**12.** Particules polymères selon la revendication 11, les particules polymères absorbant l'eau étant revêtues par au moins un aldéhyde aliphatique ou son produit de transformation avec un alcool aliphatique, une amine aliphatique, de l'ammoniac, un hypophosphite ou un phosphite.

**13.** Particules polymères selon la revendication 11 ou 12, les particules polymères absorbant l'eau contenant en outre un sulfite ou étant revêtues par un sulfite.

**14.** Particules polymères selon l'une quelconque des revendications 10 à 13, les particules polymères absorbant l'eau présentant une capacité de rétention dans une centrifugeuse d'au moins 15 g/g.

**15.** Objets hygiéniques contenant des particules polymères absorbant l'eau selon l'une quelconque des revendications 10 à 14.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 0055245 A1
- WO 2006058682 A1
- WO 2004084962 A1
- WO 2008092842 A1
- WO 2008092843 A1
- WO 2009060062 A1
- WO 03014172 A2
- WO 2004058682 A1
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 2002032962 A2
- WO 2001038402 A1
- DE 3825366 A1
- US 6241928 B
- WO 2008040715 A2
- WO 2008052971 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 2003031482 A1
- DE 3713601 A1
- EP 0640330 A1
- US 200502567575 A
- JP 1000050 A

### In der Beschreibung aufgeführte Nicht-Patentliteratur

- **F.L. BUCHHOLZ ; A.T. GRAHAM.** Modern Super-absorbent Polymer Technology. Wiley-VCH, 1998, 71-103
- Standard Test Methods for the Nonwovens Industry. 2005
- *INDA, www.inda.org*
- *Hunterlab,* 1996, vol. 8 (7), 1-4